(19) Europäisches Patentamt

European Patent Office

Office européen des brevets

(11) **EP 3 906 098 B1**

(12) **EUROPEAN PATENT SPECIFICATION**

(45) Date of publication and mention
of the grant of the patent:
**15.05.2024 Bulletin 2024/20**

(21) Application number: **20706011.2**

(22) Date of filing: **02.01.2020**

(51) International Patent Classification (IPC):
***A63B 71/08*** (2006.01)

(52) Cooperative Patent Classification (CPC):
**A63B 71/08; A61B 5/6804; A61B 5/6843;
G01L 5/0052;** A41D 13/015; A61B 2503/10;
A61B 2562/0252; A61B 2562/046;
A63B 2024/0068; A63B 2024/0071; A63B 2220/53

(86) International application number:
**PCT/GB2020/050001**

(87) International publication number:
**WO 2020/141321 (09.07.2020 Gazette 2020/28)**

(54) **SPORTS PAD WITH FORCE SENSORS**

SPORT-PAD MIT KRAFTSENSOREN

PROTECTION DE SPORT AVEC CAPTEURS DE FORCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.01.2019 GB 201900134**

(43) Date of publication of application:
**10.11.2021 Bulletin 2021/45**

(73) Proprietor: **Sportable Technologies Ltd.
London NW4 1RL (GB)**

(72) Inventor: **HUSEMEYER, Peter James Alexander
London Greater London DE11 4UB (GB)**

(74) Representative: **Gill Jennings & Every LLP
The Broadgate Tower
20 Primrose Street
London EC2A 2ES (GB)**

(56) References cited:
GB-A- 2 549 463        US-A1- 2011 260 890
US-A1- 2016 338 644    US-A1- 2017 156 667

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to a sports pad for use in estimating a force exerted by an external object on the sports pad, to a system comprising the sports pad and configured to estimate the force, and to a method for estimating a force exerted by an external object on the sports pad. In particular, the sports pad is suitable for use in contact sports or training for contact sports, and typically the sports pad will be held or worn by a person, and/or the external object that contacts the sports pad will be a part of a person or an object under the influence of a person.

### BACKGROUND TO THE INVENTION

In many sports, there is a desire to be able to capture data relating to the performance of individual players so as to monitor performance and development. In particular, in many sports, it is desirable to have data showing forces either generated or experienced by a player, often referred to as impact forces. In the present context, the forces include both static and dynamic forces that occur during sports. An example of a sport in which it is useful to have force data is rugby, in which the force exerted by a player in tackles made or when scrumming can be used to determine how effectively a player is playing, but also to establish when a force experienced is beyond the maximum considered medically safe. Other sports in which impact forces are helpful for tracking player performance, development and safety include American football, in which, again, the force of impact during tackles is of interest, and martial arts, such as taekwondo, in which the force of a fighter's strike is important.

Conventionally, impact forces have been measured in a sports context by using force transducers applied to an area of interest, or by using inertial force measurement units worn by the players. In particular, force transducers in the form of plates have been used to cover an area of interest to directly measure a force by measuring the separation of opposing surfaces of the plate. Inertial measurement units have been used in place of force transducers, but these are only able to estimate dynamic forces. This has left force transducers as the only practical way of measuring both static and dynamic forces.

Conventional force pads suffer from a number of problems. Firstly, force pads must be non-intrusive, comfortable and safe and therefore the sensors must be flexible to conform to players' bodies. Since forces are typically exerted over large areas, e.g. the entire shoulder area of a rugby player in a scrum or large areas of the body armour of an American football player in a tackle, relatively large sensors must be provided while maintaining the necessary flexibility. As has been mentioned, these large flexible sensors track the separation of the upper and lower surfaces to measure a force; however, bending a flexible sensor also causes a decrease in the separation between upper and lower surfaces and results in fictitious forces being recorded. Furthermore, repeated bending and flexing of the sensors causes an accumulation of creases, which results in further fictitious forces being measured and eventually renders the force pad unreliable and unusable. It is desirable to provide a sports pad that is able to measure forces in contact sports without the aforementioned problems.

GB2549463A discloses a wearable sports sensor for determining impact parameters. US2016338644A1 discloses smart clothing, such as a shirt or pair of pants, for ambulatory motion capture. US2011260890A1 discloses an apparatus for use in analysing the motion of a human subject including a platform upon which the subject stands. US2017156667A1 describes a wearable cranial device that has a substrate conformable to an individual's upper cranial hemisphere housing a force sensor for measuring forces associated with impacts to the exterior of the device.

### SUMMARY OF THE INVENTION

Aspects of the present invention are provided by the appended claims. Embodiments not falling in the scope of the claims are exemplary.

In accordance with a first aspect of the present invention there is provided a sports pad for use in estimating a force exerted by an external object on the sports pad, the sports pad comprising: an array of sensors, the sensors being spaced apart from one another across a grid of sensor positions of the sports pad, each sensor being configured to independently collect data indicative of the force acting on the respective sensor; and a data receiver configured to receive the collected data from each of the array of sensors such that data from a plurality of the sensors may be used to estimate a force, i.e. the force exerted by the external object on the sports pad.

By providing a sports pad with an array of spaced force sensors, it is possible to minimise the measuring of fictitious forces as the sports pad is able to bend at locations between the sensors. This will also prevent the build-up of creases in the sensors and so extend the lifetime of the product. Furthermore, again owing to the spacing of the sensors, the present sports pad does not entirely cover the area of interest with force sensitive structure and so this reduces the rate at which defective sports pads are produced during manufacture owing to defective force sensors. In contrast with sports pads with conventional force transducers, the spacing of the sensors does mean that an impact force will not be directly measured in its entirety; however, it has been found that the data indicative of force at an array of force sensor positions can be used to estimate the total force with a high degree of accuracy.

As has been mentioned above, a sports pad should be suitable for use in contact sports or training for contact sports. In particular, the sports pad will be configured to be held or worn by a person, and/or be configured for the external object to be a part of another person or an object under the influence of a person. The sports pad should therefore be sufficiently soft and/or flexible so as to not cause damage to a person holding or wearing the sports pad and/or a person striking the sports pad.

In the present context, the force estimated using the sports pad may include both dynamic and static forces. Using a rugby scrum as an example, the sports pad may be configured so as to measure an impact force on the shoulders of players as the scrum engages, and may also be configured to measure forces after the initial engagement as the scrum progresses. It will also be appreciated that the force may be generated by any relative movement of the sports pad and the external object. For example, the force may be generated by a player wearing the sports pad tackling a stationary target, or the force may be generated by an external object impacting with the sports pad as it is held or worn by a player so as to be stationary.

The sports pad comprises an array of sensors, such as force sensors. A force sensor will be understood to be a sensor that is able to gather data indicative of force, which will typically be a perpendicular force, but may include shear forces. In particular, the force sensor may directly measure a force experienced by the sensor or a pressure experienced by the sensor. Since the area of the sensor will be determined during production of the sports pad, the relationship between pressure and force for any individual sensor will be known. The force sensors are also configured to independently collect data, by which it is meant that each force sensor returns data indicative of the force experienced by that particular sensor.

The sensors will typically be arranged in a two-dimensional array across a surface of the sports pad. The sensors of the array of sensors are also spaced apart from one another. In particular, there is a gap provided between each sensor and one or more, preferably all, of its neighbouring sensors. This spaced apart arrangement deviates from some conventional force pads by sacrificing full coverage of the area of interest in exchange for benefits to overall flexibility of the sports pad, and a reduction in fictitious forces due to bending of the sensors as the sports pad may now bend at locations between the sensors. As will be described in more detail below, the invention provides that a force is estimated based on the readings of the spaced apart sensors, rather than being directly measured. It has been found that very good estimations can be achieved by fitting a continuous function to the sensor data, for example.

A data receiver is provided in the sports pad that is configured to receive the collected data from each of the array of sensors. Typically, the sensors will be connected via circuitry or wiring to the data receiver and will report their data to the data receiver for subsequent processing.

Preferably, the sensors are arranged across a substrate layer of the sports pad. The substrate is preferably a flexible substrate, such as a plastic like PET, so as to enhance the flexibility of the sports pad and prevent bending of the individual sensors. The substrate may be provided on a supporting layer of the sports pad, or may be self-supporting. Further preferably, the substrate layer defines a non-uniform grid, each sensor being located at an intersection of the grid defined by the substrate layer. That is, the substrate may define an array of apertures between grid elements in which the substrate material is absent, such that the substrate has a web-like appearance. This can reduce the weight of the sports pad, enhance flexibility and reduce manufacturing cost. As mentioned, each sensor may be located at an intersection of the grid defined by the substrate layer. This provides minimal relative movement of the sensors, as they are anchored by multiple grid elements of the substrate, and improves the accuracy of the estimated forces. In particularly preferable embodiments, the substrate provides circuitry connecting each of the sensors to the data receiver and/or powering each of the sensors. For example, printed conductive tracks may define a path across the substrate between the sensors and the data receiver.

Force pads that utilise a regular grid of sensors - square, rectangular, circular or otherwise - are prone to suffering from errors in the force measurements caused by periodicity bias when the force that is realised upon them is transmitted by an object with a periodic surface texture. For example, many athletic garments with impact protection for the wearer provided by foam, have periodic debossed lines in the foam to make them more compliant and flexible. These periodic debossed lines, when they correspond with the regular spacing of a regularly spaced array of sensors, causes erroneously low measurements. A similar problem occurs with surface textures which stand in relief. These, when aligned with the regular spacing of the regularly spaced array of sensors, causes erroneously high measurements. Therefore, the grid of sensor positions is an irregular grid of sensor positions or defines a non-uniform arrangement of the force sensors; however a regular or uniform grid may also be used. In general, an irregular or non-uniform arrangement of the sensor positions is preferable in order to prevent or minimise periodicity bias. It is common in sports for objects that may come into contact with the sports pad to have periodic surface patterns that can lead to erroneous measurements by the sports pad where the period of the surface pattern corresponds to the period of a regular force sensor grid. For example, as noted above, body armour used in contact sports may have a protective foam layer with a periodic debossing in the foam to provide it with flexibility. Should such a surface come into contact with the force sensor array, and the period of the pattern in the body armour match in any way the period of a regular grid of sensors, the sensors may, for example, take a series of force readings from the debossed areas, which would lead to the estimated force being lower that the real force of the impact. Another example of a periodic surface pattern may be the studs on a player's boots. By providing an irregular or non-uniform arrangement of the force sensors, this periodicity bias can be prevented or minimised.

Preferably the sensor positions are randomly or, more typically, pseudo-randomly arranged across the grid of sensor positions. A pseudo-random arrangement may be realised with some optimisation of the sensor positions to ensure that no gaps in the array are too large and to ensure that the sports pad retains the necessary flexibility. The resulting sports pad may have a plurality of different spacing distances between neighbouring sensors, with a greater number of different spacing distances further increasing resilience to periodicity bias. For example, at least three, preferably at least five, more preferably at least ten, most preferably at least twenty different spacing distances between neighbouring sensors may be provided across the entire sensor grid. In some embodiments, the grid may comprise regular or uniform areas and irregular or non-uniform areas where necessary to achieve a comfortable fit of the sports pad. Whether the grid is regular or irregular, the estimation of the force should take account of the relative positions of the sensors, which may be set during manufacture of the sports pad.

The sports pad comprises at least 10 sensors, preferably at least 20 sensors, more preferably at least 40 sensors. Furthermore, each sensor typically comprises a force sensitive area of at most 25 cm$^2$, preferably at most 10 cm$^2$, more preferably at most 5 cm$^2$, most preferably at most 1 cm$^2$. The advantages of the sports pad are increased when an increased number of smaller sensors are used since this provides more data points on which to base the estimation and since this provides more places for the sports pad to bend between the sensors. Furthermore, smaller sensors will reduce the likelihood of bending and so tend to minimise the measuring of fictitious forces.

Each sensor comprises a force sensitive area and the total force sensitive area of the array of sensors is at most 50%, more preferably at most 30%, further preferably at most 20%, of the total area covered by the array of sensors. Again, the present sports pad does not aim to directly read the force experienced, but rather provides data points across the area of interest that may be used to accurately estimate the force. This change in approach means that a relatively small proportion of the surface area of the sports pad may be made up by the sensors. As has been explained above, this improves the flexibility and comfort of the sports pad. Furthermore, a lower area of coverage, for any particular sensor size, reduces the number of individual sensors required. This increases the manufacturing yield for the sports pad as the probability of one or more sensors in the sports pad being defective decreases. Further still, a decrease in the number of sensors will reduce the amount of data that needs to be processed and therefore reduce cost and complexity of implementing the sports pad.

Preferable combinations of the above options include a sports pad with at least 20 sensors, each with a force sensitive area of at most 10 cm$^2$ and/or with a total force sensitive area that is at most 50% of the total area covered by the array of sensors. An even more preferable combination would be a sports pad with at least 40 sensors, each with a force sensitive area of at most 5 cm$^2$ and/or with a total force sensitive area that is at most 30% of the total area covered by the array of sensors. One practical implementation that has been found to give very good results comprises at least 80 sensors, with a force sensitive area of at most 1 cm$^2$ and with a total force sensitive area that is at most 20% of the total area covered by the array of sensors.

It should be noted that a move towards a smaller total force sensitive area of the sports pad, i.e. providing fewer sensors and with smaller force sensitive areas, increases the benefit of an irregular arrangement of the force sensors. This is because, as the amount of force sensitive area is reduced, periodicity bias will tend to have a greater effect on the estimated force, i.e. since the force estimated by the sensors will be extrapolated over a greater area of the sports pad. Therefore, particularly preferred embodiments utilise an irregular array of force sensors in combination with a relatively low number of sensors and/or a relatively low total force sensitive area.

As has been mentioned above, the sensors will typically measure either force or pressure; however, preferably each sensor is configured to directly collect the force acting on the respective sensor.

In many embodiments, the sports pad comprises a data transmitter configured to transmit the data received by the data receiver and preferably being configured to wirelessly transmit the data. The data transmitter will typically be configured to transmit the data to a data receiver of an external electronics unit, such as a computer.

As has been mentioned above, the sports pad will typically comprise one or more padded layers arranged over at least one side of the array of sensors so as to prevent damage to the sensors and injury to players. A padded layer arranged over a top surface of the array of sensors would protect the external object, which may be a person, from the sensors, while a padded layer arranged over a bottom surface of the array of sensors would protect a wearer from the sensors.

While the sports pad could be provided across a planar surface, for example, across the surface of a tackle pad, preferably the sports pad is configured to be fitted to a non-planar surface, such as the body of a player, such that the array of sensors defines a non-planar force sensing layer.

The sports pad will typically be incorporated in a sports item, such as body armour, sports clothing, or sports training equipment. The sports pad may be removable or may be integral to the sports item. In particular, preferably the sports pad is incorporated in a wearable sports item, such as a sports shirt or vest, and the array of sensors extends over an area corresponding to one or more of a wearer's shoulders, arms, sternum, abdomen, sides, upper back, left middle back, right middle back and lower back.

In accordance with a second aspect of the present invention, there is provided a system configured to estimate a force exerted by an external object on a sports pad, the system comprising: a sports pad according to the first aspect of the invention; and a processor configured to calculate an estimated force based on the data collected by a plurality of the sensors.

Typically the processor will be part of an external electronics unit, such as a computer. Where the sports pad has a data transmitter for transmitting the collected data, preferably the system comprises a complementary receiver configured to receive the data transmitted by the data transmitter, the processor being configured to process the data received by the complementary receiver. For example, the complementary receiver may receive the data from the sports pad and write the data to memory, which is then accessed by the processor.

Preferably, the processor is configured to calculate an estimated force by fitting a continuous function to the data collected by the plurality of sensors. That is, the data may indicate the force or pressure at a plurality of points across the two-dimensional array; however, the force that caused those data readings will likely have been a large object exerting a continuous force profile. Therefore, the processor may fit a continuous function to the force point values across the array, effectively interpolating force or pressure values between the sensors so as to arrive at a surface fit that should closely correspond to the force profile of the impact that caused the force readings. Here, we are essentially performing a surface fit to the data so as to allow us to estimate the total force by calculating the area beneath this surface. Any surface fit may be used, such as a two-dimensional spline interpolation, to determine the continuous function. This has been found to provide a very accurate estimate of the total force applied. Alternatively, a more crude method may, for each sensor, assume the measured force is constant across an area associated with that sensor and, on that basis calculate the force across the sensor area. The total force would then be estimated by summing the forces for each sensor area across the sports pad. This would essentially be a nearest-neighbour interpolation of the data.

Where the processor fits a continuous function to the data, preferably, the processor is further configured to calculate an estimated force by calculating the integral of the fitted continuous function. This has been found to achieve particularly good estimations of forces in practice.

In all of the above embodiments, preferably the processor is configured to calculate an estimated force based on the data collected by a plurality of the sensors and based on a predetermined spacing of the plurality of sensors. That is, the calculation operates based on a known expected positioning of each of the sensors. For example, the sensors may be known to be spaced at 5 cm intervals on a regular square grid. While preferable, it is foreseen that other methods could be used. For example, the sensors could report relative positioning as part of their data set so as to account for movement of the sensors away from their position following manufacture.

In some embodiments, the data from all of the sensors may be used to estimate the total force across the whole array of sensors. However, in other embodiments, the processor is configured to select data from a subset of the plurality of sensors and to calculate an estimated force for a subarea corresponding to the selected subset based on the data collected by said subset of the plurality of sensors. This may allow the system to estimate forces in discrete subareas of the sports pad. These subsets may be predetermined. For example, if the sports pad extends across a wearer's shoulders, the system may be configured with a left-shoulder subarea and a right shoulder subarea. A certain subset of the plurality of sensors would be specified as being in the left-shoulder subarea and another subset of the plurality of sensors would be specified as being in the right-shoulder subarea. The system may then estimate a force exerted on the left-shoulder area and/or the right-shoulder area by using only the data from the corresponding subset of the plurality of sensors. For example, the system may fit a continuous function to the data points from the corresponding subset of the plurality of sensors and then calculate the integral of this function to determine the force exerted across that subset. Alternatively, for a sports pad worn in rugby across a wearer's upper body and shoulders, the sensors may be divided into upper subareas (i.e. above the armpit) and lower subareas (i.e. below the armpit), with forces in the upper areas being used to identify high tackles.

While the subareas may be predetermined, they could also be determined on the basis of the forces measured by the sensors. That is, the subset of the plurality of sensors may be selected based on the data collected by the plurality of sensors. The subset of the plurality of sensors may be selected by comparing the data collected by each sensor with a reference value, or by comparing the data collected by each sensor with the data collected by one or more other sensors. For example, a subset of sensors may be selected based on sensors that detect a force rate of change exceeding a predetermined threshold value or that neighbour such a sensor. Alternatively, for example, a subset of sensors may be selected based on sensors that detect a force exceeding a predetermined threshold value or that neighbour a sensor detecting a force exceeding that threshold. The system may then estimate a force exerted on the selected subarea by using only the data from the corresponding subset of the plurality of sensors. This may have a number of advantages.

This allows the system to ignore the data from sensors that are far removed from a main point of impact, which could inflate the estimated force of a particular impact. For example, a rugby player gripping the ball against the force pad themselves who is then tackled is experiencing two simultaneous forces and if it is desired to estimate the force of the tackle experienced, then this could be overestimated if the gripping of the ball is added to the total force. This may also reduce the processing burden for estimating a force and/or may allow a series of forces to be estimated across the subarea at a higher frequency, i.e. with a smaller interval between successive force measurements. In other words, the processor may configured to repeat the calculation of estimated force collect data from the subset of the plurality of sensors at faster rate than data is collected from sensors outside of the subset of the plurality of sensors. The subset may change dynamically as the force profile evolves by continuously sampling all sensors, but the selected subset corresponding to the highest force may be sampled more frequently to provide the highest resolution on only those areas of greatest interest.

The ability to isolate discrete areas of the sports pad and estimate the force in that region has advantages across sports disciplines. For example, it would be possible to measure forces in an injury prone area for an athlete and look at the force experienced by just that area to establish whether technique is contributing to injury. Alternatively, for athletes returning from injury, it would be possible to identify subareas at the site of injury so as to monitor the site for forces that may impede the recovery process. As yet another example, it would be possible to divide each shoulder into front, middle and back areas to analyse a player's tackling technique, scrum technique or scrimmage technique in rugby and American football.

In the above examples, the subsets of the sensors are predetermined, e.g. left-shoulder subarea; however, this is not essential. The processor may also been configured to automatically identify a subarea of interest and use the data from the automatically selected subset of sensors. For example, the processor may be configured to identify a subarea that experiences a high force of impact. In this example, the processor may select only sensors that collected force data over some predetermined or automatically determined threshold value and then estimate a force based on the data from that automatically selected subset. This may allow for peak forces to be monitored and may help identify areas that could have been injured in an event.

In all of the above embodiments, preferably the system is configured to provide an output based on the estimated force. For example, the system may output the estimated force to a user by displaying the estimated force on a display. Alternatively, the system may provide an alert, such as an audio or visual alert, if a force exceeds a predetermined threshold value, which may be a value above which it is desirable to evaluate the player on medical or safety grounds. The system may alternatively be used to monitor a change in force experienced over time.

Preferably, the processor is configured to repeat the calculation of estimated force using data collected by a plurality of sensors at irregular or non-uniform time intervals, preferably at random or pseudo-random time intervals. Using data from the force sensors at irregular or non-uniform time intervals prevents or minimises periodicity bias in a series of force measurements. For example, if a force was being applied to the sports pad over a period of time in a periodic manner, as may occur if, for example, the period of a player's running stride affects how they impart force into a player being tackled, a periodic sampling interval of the force sensors may lead to each force estimate being above or below the average force across the duration of the tackle. By sampling at irregular or non-uniform time intervals, this periodicity bias may be reduced or eliminated.

The system may also comprise a second sports pad according to the first aspect of the invention and a processor configured to calculate an estimated force based on the data collected by a plurality of the sensors of the second sports pad, the processor or processors preferably being configured to compare the data collected by the plurality of sensors of the first sports pad with the data collected by the plurality of sensors of the second sports pad, to identify an event occurring between the two sports pads based on said comparison, and to output an indication of the event occurring between the two sports pads. The processor may be the same processor that calculates the estimated force for the first sports pad or may be a different processor. The comparison may involve estimating force using the techniques described above and comparing the magnitude of an estimated force of the first sports pad and the magnitude of an estimated force of the second sports pad and/or a time of the estimated force of the first sports and a time of the estimated force of the second sports pad. Similar forces may indicate a high probability of the sports pads being involved in the same event, e.g. one player tackling the other, just as the forces occurring at the same time may indicate a high probability of the sports pads being involved in the same event. Preferably both magnitude and time are compared simultaneously. This process is capable of, in real-time, finding force histories between players which correlate strongly. This may allow the system, for example, to determine in real-time the victim of a high-tackle and the perpetrator.

In accordance with a third aspect of the present invention, there is provided a method of estimating a force exerted by an external object on a sports pad, the method comprising: collecting, using an array of sensors, data indicative of the force acting on each of the respective sensors, wherein the sensors are spaced from one another across a grid of sensor positions within the sports pad, and wherein each sensor is configured to independently collect the data indicative of force; receiving, at a data receiver, the collected data from each of the array of sensors; calculating, using a processor, an estimated force based on the data collected by a plurality of the sensors.

As with the above system, preferably the step of calculating the estimated force comprises fitting a continuous function to the data collected by the plurality of sensors. Further preferably, calculating the estimated force comprises calculating an integral of the fitted continuous function.

Again, calculating the estimated force is preferably based on the data collected by a plurality of the sensors and based on a predetermined spacing of the plurality of sensors.

Furthermore, as described above with regard to the second aspect of the invention, the method may comprise selecting a subset of the plurality of sensors and calculating an estimated force for a subarea corresponding to the selected subset based on the data collected by said subset of the plurality of sensors.

It will be appreciated that the method according to the third aspect of the present invention may be performed using a sports pad according to the first aspect of the invention and/or with a system according to the second aspect of the present invention.

It will be clear from the above that there are numerous advantageous associated with measuring a force using an array of individual, spaced sensors in place of a complete covering of force detecting surface. As has been mentioned, the above configuration leads to a reduction in the measuring of fictitious forces and extends the operating life of the sports pad. It also makes the sports pad more flexible and therefore more comfortable for a wearer, and more appropriate for safe use in contact sports.

## BRIEF DESCRIPTION OF THE DRAWINGS

The present invention will now be explained with reference to the Figures, of which:

Figure 1 shows a prior art sports pad in a top view;

Figures 2A to 2C show a sports pad according to a first embodiment of the invention in a top view and a portion of the sports pad in an enlarged top view and an enlarged cross-section view respectively;

Figure 3 shows, schematically, a system integrating the sports pad according to the first embodiment; and

Figure 4 is a flow diagram illustrating a method of using the system of Figure 3 to estimate a force exerted by an external object on the sports pad.

## DETAILED DESCRIPTION

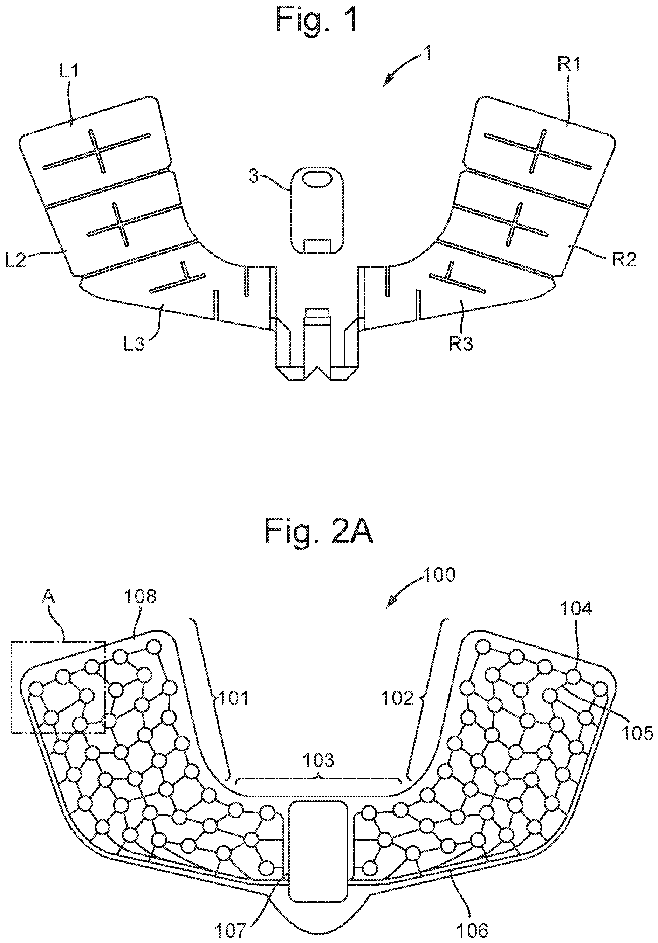

Figure 1 shows a prior art force pad 1 for measuring the force exerted by an external object on the force pad. Specifically, Figure 1 shows a generally U-shaped shoulder pad configured to sit on a wearer's shoulders, with two shoulder covering portions connected by a central portion that extends over a wearer's upper back.

The prior art force pad 1 shown in Figure 1 comprises six flexible sensors R1 to R3 and L1 to L3, with three on either side. When fitted, each shoulder thereby has a first sensor R1, L1 positioned on the front of the shoulder, a second sensor R2, L2 abutting the first sensor and positioned on the top of the shoulder and a third sensor R3, L3 abutting the second sensor and positioned on the back of the shoulder and extending towards the centre of the wearer's upper back. Each of these flexible sensors is connected via wiring 2 to an electronics unit 3 arranged to sit approximately between the shoulder blades of the wearer.

Together, the flexible sensors R1 to R3 and L1 to L3 substantially cover the shoulders of the wearer. Each of the flexible sensors R1 to R3 and L1 to L3 in the prior art force pad directly measures a force acting on the respective sensor by monitoring the separation of the upper and lower surfaces of the sensor. Together, the sensors are able to measure a force over the complete shoulder area of the wearer. However, as will be appreciated from Figure 1, each of the flexible sensors R1 to R3 and L1 to L3 is required to bend to match the contours of the wearer's shoulders. This bending of the sensors also causes a decrease in the separation between upper and lower surfaces, leading to the reading of fictitious forces. Furthermore, wear-and-tear on flexible sensors (through tackles made, scrumming etc.) causes accumulation of creases which results in further fictitious forces being recorded and can ultimately lead to the force pad to cease working.

Figures 2A to 2C show a sports pad 100 according to the present invention. The sports pad 100 is again a generally U-shaped shoulder pad configured to sit on a wearer's shoulders, with two shoulder-covering portions 101, 102 connected by a central portion 103 that extends over a wearer's upper back. The sports pad thus takes a similar profile to a yoke.

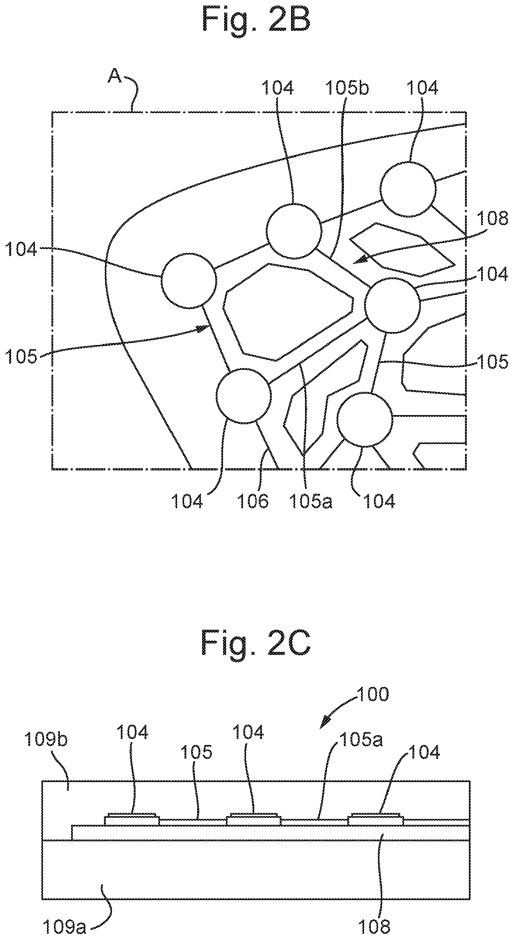

The sports pad 100 comprises an array of spaced apart force sensors 104 arranged across the two shoulder-covering portions 101, 102 and extending into the central portion 103. In particular, the force sensors 104 are arranged across a flexible PET substrate layer defining an open grid, with each force sensor being located at a junction of the grid defined by the substrate. The spacing of the force sensors is non-uniform across the array of force sensors in order to provide an irregular or non-uniform arrangement of the sensors, which is less susceptible to periodicity bias for the reasons described above. That is, as shown in Figure 2B, which corresponds to area A of the sports pad in Figure 2A, each force sensor is positioned at a junction between multiple grid elements of the grid defined by the substrate layer. This grid arrangement reduces the weight of the sensor layer since it provides apertures through the substrate layer 108. In this embodiment, the substrate also comprises printed circuitry 105 in the form of printed silver for transmitting power to the sensors and transmitting the data collected by the force sensors. The force sensors 104 are arranged in a plurality of rows along the sports pad and the grid defined by the substrate 108 generally extends along the rows and between neighbouring sensors in adjacent rows, i.e. generally along columns of the array.

The printed circuitry 105a extending along the rows of the array supplies power to each of the plurality of sensors 104. This printed circuitry joins a main circuitry path 106 at the end of each row, which then extends around the periphery of the sports pad to the electronics unit 107. Meanwhile, the printed circuitry 105b extending between the rows, i.e. generally along the columns of the array, provides the data paths along which data from each of the force sensors 104 is transferred to the electronics unit 107.

Suitable force sensors include the FlexiForce A101 Sensor sold by Tekscan, Inc. of 307 West First Street, South Boston, MA 02127, United States.

As most clearly shown in Figure 2C, the array of force sensors 104 are distributed across the substrate layer 108 and are interconnected by the printed circuitry. An inner padded layer 109a is provided beneath the substrate layer 108 to separate and cushion the force sensors and substrate layer 108 relative to the wearer's shoulders. An outer padded layer 109b is also provided over the array of force sensors to shield the force sensors from the environment and to cushion any impacting object, which may be another person. Preferably the substrate layer 108, inner padded layer 109a and outer padded layer 109b are also waterproof, to protect the force sensors 104.

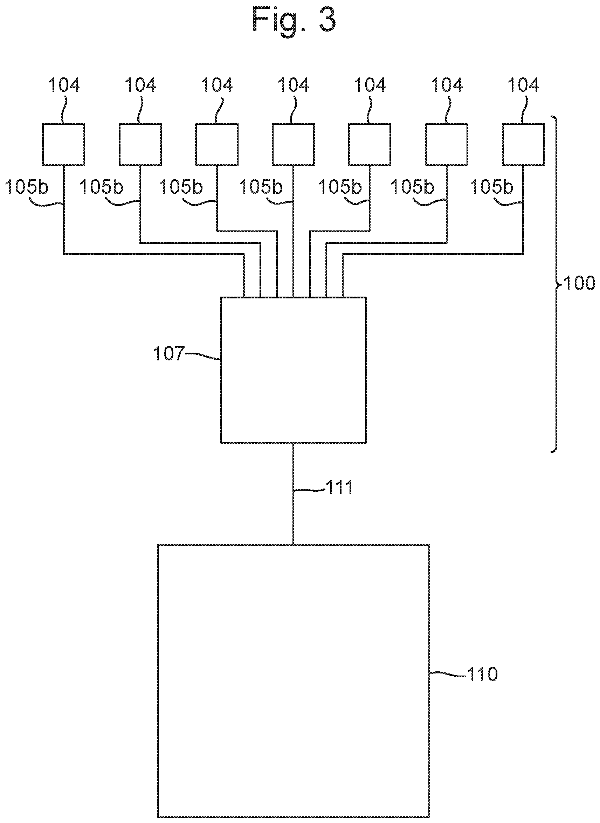

Figure 3 shows, schematically, a system for estimating a force exerted by an external object on the sports pad. Figure 3 shows, schematically, a sports pad 100, which may be the sports pad shown in Figures 2A to 2C. Figure 3 shows a plurality of force sensors 104 connected via circuitry 105b to an electronics unit 107. In this Figure, only seven force sensors are shown for simplicity; however, it will be appreciated, especially in view of Figure 2A, that many more sensors are typically connected to the electronics unit 107. The electronics unit 107 may comprise a memory module for storing data received from the force sensors. This is particularly suitable where the sports pad is configured to collect data relating to a session, before the data is transferred to an external electronics unit 110 after the session for evaluating impact forces relating to the entire session. This may be performed by removing the memory module, which may for example be a memory card. The electronics unit may, alternatively, or in addition, comprise a data transmitter configured to transmit data to a receiver of an external electronics unit. Figure 3 shows a connection 111 between the electronics unit 107 and the external electronics unit 110. The data transmitter may utilise a hardware port, such as a USB port, to connect the electronics unit 107 to the external electronics unit 110. However, preferably, the data transmitter uses a wireless data transmission means, for example a Bluetooth transmitter or radio transmitter. A wireless transmitter, as used in this preferred embodiment, may allow for data to be transmitted in real-time, which is preferable for broadcast sports. A small power supply is also provided within the external electronics unit 110 to power the components on the sports pad. This may be, for example, a battery module comprising one or more replaceable or rechargeable batteries.

The external electronics unit 110 may be, for example, a computer, having a processor, e.g. a central processing unit (CPU), hard drive, random access memory (RAM), display and one or more input devices. Where the electronics unit 107 comprises a data transmitter, preferably the external electronics unit has a complementary data receiver for establishing a direct data connection 111 between the electronics unit 107 of the sports pad and the external electronics unit 110.

While the embodiment of Figure 3 uses an external electronics unit 110 to carry out the estimation of force experienced, in alternative embodiments, the electronics unit 107 on the sports pad may comprise a processor and the electronics unit 107 may thereby be adapted to estimate a force directly on the sports pad.

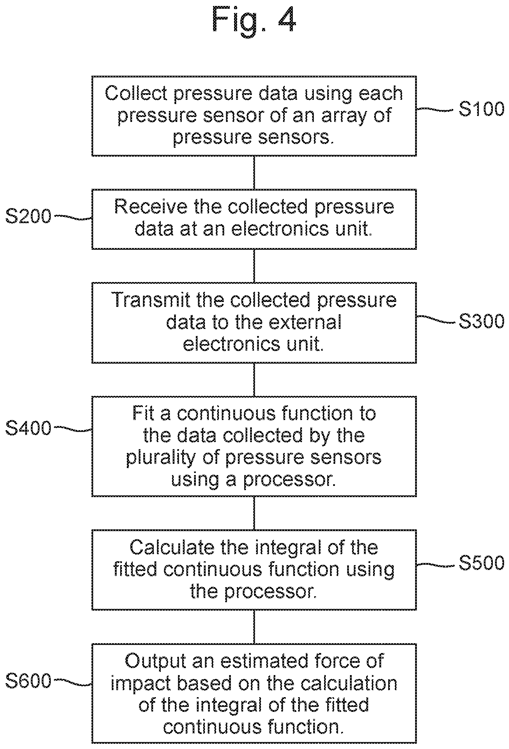

A method of using the system shown in Figure 3 will now be described with reference to Figure 4, which is a flow diagram showing the steps taken to estimate the impact force of an object with the sports pad.

The sports pad 100 is fitted to a wearer who is to engage in a sports activity in which one or more external objects will come into contact with the sports pad. In step S100, the sports pad collects force data using each force sensor 104 of the array of force sensors. The collection of the force data captures data relating to an increase in force exerted on a plurality of the force sensors by an external object.

In step S200, the force data is received at the electronics unit 107, having been transmitted to the electronics unit 107 via the circuitry 105b.

In step S300, the collected force data received by the electronics unit 107 is transmitted, e.g. wirelessly, to an external electronics unit 110, such as a computer.

The external electronics unit 110 then fits a continuous function to the data collected by the plurality of force sensors using a processor in step S400. One way of fitting a continuous function to the data will now be described in detail.

Each of the sensors can be labelled with an index i. The coordinates of each sensor are fixed relative to one another by their positioning on the substrate layer 108 at the junctions of the grid. Their coordinates can be expressed as $(x_i, y_i)$ while the force output for each sensor, can be expressed as $f_i$. As has been mentioned above, the surface area of the force sensors is fixed and so the sensor can be adapted to either measure a pressure, which can be used to determine the force in combination with the surface area, or directly measure a force. The resulting data set will comprise, for each force sensor, the coordinate data of the force sensor in the array, and the force data collected by the sensor. The resulting data set, for a specific time t, may therefore be represented as

$$[(x_1, y_1, f_1), \dots (x_n, y_n, f_n).]$$

where n is the number of sensors in the array.

The surface form must be specified *a priori*. The form may be selected as desired to balance the accuracy of the estimate with the computational power required to process higher surface forms. To fit a surface of degree two, one needs to determine the coefficients $a_{jk}$ in the equation

$$g(x, y) = a_{20}x^2 + a_{11}xy + a_{02}y^2 + a_{10}x + a_{01}y + a_{00}$$

If there are exactly six sensors, with six force outputs, then this surface can be determined exactly. However, in the above sports pad, there are more sensors than there are coefficients in the equation, and therefore the coefficients are overconstrained. In order to determine the coefficients in the overconstrained scenario, construct an error function and to then minimize it with respect to the coefficients. The present method minimizes the sum of the square errors.

Let the error function be

$$E = \sum_i [g(x_i, y_i) - f_i]^2$$

Now, the six equations for the six coefficients can be constructed by taking the partial derivative of the error function with respect to each of the coefficients and setting it equal to zero. This approach allows us to determine the coefficients which minimize the error function, or make it stationary for small perturbations in the coefficients.

$$\frac{\partial E}{\partial a_{jk}} = 2 \sum_i [g(x_i, y_{i)} - f_i][\frac{\partial g(x, y)}{\partial a_{jk}}] = 0$$

These equations can be arranged into a matrix as follows

$$\begin{bmatrix} \sum x_i^4 & \sum x_i^3 y_i & \sum x_i^2 y_i^2 & \sum x_i^3 & \sum x_i^2 y_i & \sum x_i \\ \sum x_i^3 y_i & \sum x_i^2 y_i^2 & \sum x_i y_i^3 & \sum x_i^2 y_i & \sum x_i y_i^2 & \sum x_i y_i \\ \sum x_i^2 y_i^2 & \sum x_i y_i^3 & \sum y_i^4 & \sum x_i y_i^2 & \sum y_i^3 & \sum y_i^2 \\ \sum x_i^3 & \sum x_i^2 y_i & \sum x_i y_i^2 & \sum x_i^2 & \sum x_i y_i & \sum x_i \\ \sum x_i^2 y_i & \sum x_i y_i^2 & \sum y_i^3 & \sum x_i y_i & \sum y_i^2 & \sum y_i \\ \sum x_i^2 & \sum x_i y_i & \sum y_i & \sum x_i & \sum y_i & N \end{bmatrix} \begin{Bmatrix} a_{20} \\ a_{11} \\ a_{02} \\ a_{10} \\ a_{01} \\ a_{00} \end{Bmatrix} = \begin{bmatrix} \sum x_i^2 f_i \\ \sum x_i y_i f_i \\ \sum y_i^2 f_i \\ \sum x_i f_i \\ \sum y_i f_i \\ \sum f_i \end{bmatrix}$$

or in a more compact notation as

$$\left[\sum\right]\{a_{jk}\} = \{b\}$$

The coefficients can now be determined by premultiplying both sides by the inverse

$$\{a_{jk}\} = \left[ \sum \right]^{-1} \{b\}$$

The total force F can then be estimated in step S500 by integrating $g(x_i, y_i)$ over the total area $A$ and then dividing by the total area as follows:

$$F = \frac{\int g(x, y) dA}{\int dA}$$

The total force may then be output in step S600. For example, the force may be displayed on a screen of the external electronics unit 110. It should be noted here that any surface fit, for example a two-dimensional spline interpolation, may be used according to the present invention to determine the continuous function and ultimately arrive at the total force output in step S600.

This process may be repeated over time in order to monitor the development of an applied force. As explained above, the the force output for each sensor $f_i$ may be sampled at irregular or non-uniform time intervals and used in successive force estimations to remove periodicity bias in the monitoring of the development of an applied force to give a truer representation of the development of the applied force.

While the above process describes estimating the force across the entire sensor array, it will be appreciated that it can equally be applied to subsets of the array. As explained above, the subsets may be predetermined, e.g. subsets may be programmed in that correspond to areas of interest of the sports pad, such as certain anatomical regions of a player. Alternatively, the subset may be selected dynamically. In one instance, all sensors detecting a pressure over a predetermined value may be selected to define the subarea and the above calculations adapted for that dynamically selected subset.

This process may be performed for multiple events occurring at different times and on different sports pads used in the system in order to estimate the force of impact of a number of different objects over a training or playing session, for example. Where multiple sports pads are used in the system, the above calculation processes may be run simultaneously and in parallel for each sports pad.

This information can be valuable in training, for example, to measure the strength of a tackle and track players who are improving or deteriorating over time. The information can also be used to compare static and dynamic forces, e.g. how much force a player imparts when a scrum engages compared with how much they are able to impart when the scrum is underway. The information can be medically useful in determining the forces imposed on players in play. If the values exceed those which are believed to be acceptable then players can be removed from the field of play to avoid injury, rules can be modified or additional protective equipment can be introduced. Forces can also be tracked over time to monitor player fatigue, which can be helpful for deciding when a player should be rested, but also monitoring player development, e.g. after how long fatigue begins to set in. The information may also be of great interest to spectators, especially those not physically present at the event who watch the event as a broadcast, for example television broadcast or a webcast. In this case, not all of the collected information need be displayed to the spectators but a selection which can be selected by the end user or by an editor of the broadcaster. Thus in the first case an individual end user could choose to monitor the performance of a favourite player. In the second case an editor of the broadcaster, for example seeing a player about to be tackled, could choose to broadcast the forces acting on that player.

While the invention has been described by reference to a shoulder pad for use in rugby, it will be appreciated that the sports pad can be used in many different sports contexts. For example, the sports pad could be provided on the exterior of body armour used in American football for example, or used in training items, such as tackle shields. The sports pad could also be integrated across a full shirt so to provide data concerning forces measured across the whole upper body of a wearer.

**Claims**

1. A sports pad (100) for use in estimating a force exerted by an external object on the sports pad, the sports pad comprising:

    a two-dimensional array of sensors (104), the array of sensors comprises at least 10 sensors, the sensors being spaced apart from one another across a grid of sensor positions of the sports pad, each sensor being configured to independently collect data indicative of the force acting on the respective sensor;
a data receiver (107) configured to receive the collected data from each of the array of sensors such that data from a plurality of the sensors may be used to estimate a force,
**characterised in that**:

the grid of sensor positions is a non-uniform grid of sensor positions; and
each sensor comprises a force sensitive area and wherein the total force sensitive area of the array of sensors is at most 50% of the total area covered by the array of sensors.

2. A sports pad according to claim 1, wherein the sensors are arranged across a substrate layer (108) of the sports pad, wherein preferably the substrate layer defines a grid, each sensor being located at an intersection of the grid defined by the substrate layer.

3. A sports pad according to any of the preceding claims, wherein sensor positions are randomly or pseudo-randomly arranged across the grid of sensor positions.

4. A sports pad according to any of the preceding claims, wherein each sensor is configured to directly collect the force acting on the respective sensor, and/or wherein each sensor comprises a force sensitive area of at most 25 cm$^2$, preferably at most 10 cm$^2$, more preferably at most 5 cm$^2$, most preferably at most 1 cm$^2$.

5. A sports pad according to any of the preceding claims, further comprising a data transmitter configured to transmit the data received by the data receiver and preferably being configured to wirelessly transmit the data.

6. A sports pad according to any of the preceding claims, wherein the sports pad is configured to be fitted to a non-planar surface such that the array of sensors defines a non-planar sensing layer.

7. A sports pad according to any of the preceding claims, wherein the sports pad is incorporated in a sports item, such as body armour, sports clothing, or sports training equipment, wherein preferably the sports pad is incorporated in a wearable sports item, and wherein the array of sensors are arranged to extend over an area corresponding to one or more of a wearer's shoulders, arms, sternum, abdomen, sides, upper back, left middle back, right middle back and lower back.

8. A system configured to estimate a force exerted by an external object on a sports pad, the system comprising:

a sports pad according to any of claims 1 to 7;
a processor configured to calculate an estimated force based on the data collected by a plurality of the sensors.

9. A system according to claim 8, wherein the processor is configured to calculate an estimated force by fitting a continuous function to the data collected by the plurality of sensors, wherein preferably the processor is configured to calculate an estimated force by calculating the integral of the fitted continuous function.

10. A system according to any of claims 8 to 9, wherein the processor is configured to select data from a subset of the plurality of sensors and to calculate an estimated force for a subarea corresponding to the selected subset based on the data collected by said subset of the plurality of sensors, wherein preferably the subset of the plurality of sensors is selected based on the data collected by the plurality of sensors, and wherein further preferably the subset of the plurality of sensors is selected by comparing the data collected by each sensor with a reference value, or by comparing the data collected by each sensor with the data collected by one or more other sensors, and/or wherein further preferably the processor is configured to repeat the calculation of estimated force and wherein data is collected from the subset of the plurality of sensors at faster rate than data is collected from sensors outside of the subset of the plurality of sensors.

11. A system according to any of claims 8 to 10, wherein the processor is configured to repeat the calculation of estimated force using data collected by a plurality of sensors at non-uniform time intervals, preferably at random or pseudo-random time intervals.

12. A system according to any of claims 8 to 11, wherein the sports pad is a first sports pad and further comprising a second sports pad according to any of claims 1 to 7 and a processor configured to calculate an estimated force based on the data collected by a plurality of the sensors of the second sports pad, the processor or processors being configured to compare the data collected by the plurality of sensors of the first sports pad with the data collected by the plurality of sensors of the second sports pad, to identify an event occurring between the two sports pads based on said comparison, and to output an indication of the event occurring between the two sports pads, wherein preferably identifying the event occurring between the two sports pads is based on a comparison of the magnitude of an estimated force of the first sports pad and the magnitude of an estimated force of the second sports pad and/or a time of the estimated force of the first sports and a time of the estimated force of the second sports pad.

13. A method of estimating a force exerted by an external object on a sports pad, the method comprising:

collecting (S100), using a two-dimensional array of sensors, the array comprising at least 10 sensors, data indicative of the force acting on each of the respective sensors, wherein the sensors are spaced from one another across a grid of sensor positions within the sports pad, and wherein each sensor is configured to independently collect the data indicative of force;
receiving (S200), at a data receiver, the collected data from each of the array of sensors;
calculating, using a processor, an estimated force based on the data collected by a plurality of the sensors;
**characterised in that**:

the grid of sensor positions is a non-uniform grid of sensor positions; and
each sensor comprises a force sensitive area and wherein the total force sensitive area of the array of sensors is at most 50% of the total area covered by the array of sensors.

14. A method according to claim 13, wherein the step of calculating the estimated force comprises fitting (S400) a continuous function to the data collected by the plurality of sensors, wherein preferably the step of calculating the estimated force comprises calculating (S500) an integral of the fitted continuous function.

15. A method according to any of claims 13 to 14, wherein calculating the estimated force is based on the data collected by a plurality of the sensors and based on a predetermined spacing of the plurality of sensors and/or comprising selecting a subset of the plurality of sensors and calculating an estimated force for a subarea corresponding to the selected subset based on the data collected by said subset of the plurality of sensors.

**Patentansprüche**

1. Sportpolster (100) für die Verwendung bei der Abschätzung einer Kraft, die durch ein äußeres Objekt auf das Sportpolster ausgeübt wird, wobei das Sportpolster Folgendes umfasst:

eine zweidimensionale Reihe von Sensoren (104), wobei die Reihe von Sensoren mindestens 10 Sensoren umfasst, wobei die Sensoren über ein Raster von Sensorpositionen des Sportpolsters voneinander beabstandet sind, wobei jeder Sensor konfiguriert ist, um unabhängig Daten zu sammeln, die die Kraft, die auf den jeweiligen Sensor wirkt, anzeigen;
einen Datenempfänger (107), der zum Empfang der gesammelten Daten von jedem der Reihe von Sensoren konfiguriert ist, sodass Daten von einer Vielzahl der Sensoren zur Abschätzung einer Kraft verwendet werden können,
**dadurch gekennzeichnet, dass**:

das Raster von Sensorpositionen ein ungleichmäßiges Raster von Sensorpositionen ist; und
jeder Sensor eine kraftempfindliche Fläche umfasst und wobei die gesamte kraftempfindliche Fläche der Reihe von Sensoren höchstens 50 % der Gesamtfläche beträgt, die durch die Reihe von Sensoren bedeckt ist.

2. Sportpolster nach Anspruch 1, wobei die Sensoren über eine Substratschicht (108) des Sportpolsters angeordnet sind, wobei bevorzugt die Substratschicht ein Raster definiert, wobei sich jeder Sensor an einem Schnittpunkt des Rasters befindet, das durch die Substratschicht definiert ist.

3. Sportpolster nach einem der vorstehenden Ansprüche, wobei Sensorpositionen über das Raster von Sensorpositionen zufällig oder pseudozufällig angeordnet sind.

4. Sportpolster nach einem der vorstehenden Ansprüche, wobei jeder Sensor zum direkten Sammeln der Kraft, die auf den jeweiligen Sensor wirkt, konfiguriert ist und/oder wobei jeder Sensor eine kraftempfindliche Fläche von höchstens 25 cm$^2$, bevorzugt höchstens 10 cm$^2$, bevorzugter höchstens 5 cm$^2$, am bevorzugtesten höchstens 1 cm$^2$ umfasst.

5. Sportpolster nach einem der vorstehenden Ansprüche, das weiter einen Datenüberträger umfasst, der zur Übertragung der Daten, die von dem Datenempfänger empfangen werden, konfiguriert ist und bevorzugt zur drahtlosen Übertragung der Daten konfiguriert ist.

6. Sportpolster nach einem der vorstehenden Ansprüche, wobei das Sportpolster zur Anpassung an eine nichtplanare Oberfläche konfiguriert ist, sodass die Reihe von Sensoren eine nichtplanare Sensorschicht definiert.

7. Sportpolster nach einem der vorstehenden Ansprüche, wobei das Sportpolster in einem Sportartikel, wie zum Beispiel Körperschutz, Sportbekleidung oder Sporttrainingsausrüstung, eingebaut ist, wobei bevorzugt das Sportpolster in einem tragbaren Sportartikel eingebaut ist und wobei die Reihe von Sensoren angeordnet sind, um sich über eine Fläche zu erstrecken, die einem oder mehreren eines Trägers Schultern, Arme, Sternums, Bauchbereichs, Seiten, oberen Rückens, linken mittleren Rückens, rechten mittleren Rückens und unteren Rückens entspricht.

8. System, das zur Abschätzung einer Kraft konfiguriert ist, die durch ein äußeres Objekt auf ein Sportpolster ausgeübt wird, wobei das System Folgendes umfasst:

ein Sportpolster nach einem der Ansprüche 1 bis 7;
einen Prozessor, der zur Berechnung einer abgeschätzten Kraft basierend auf den Daten, die durch eine Vielzahl der Sensoren gesammelt werden, konfiguriert ist.

9. System nach Anspruch 8, wobei der Prozessor zur Berechnung einer abgeschätzten Kraft durch Anpassen einer kontinuierlichen Funktion an die Daten, die durch die Vielzahl von Sensoren gesammelt werden, konfiguriert ist, wobei bevorzugt der Prozessor zur Berechnung einer abgeschätzten Kraft durch Berechnung des Integrals der angepassten kontinuierlichen Funktion konfiguriert ist.

10. System nach einem der Ansprüche 8 bis 9, wobei der Prozessor zur Auswahl von Daten aus einer Untergruppe der Vielzahl von Sensoren und zur Berechnung einer abgeschätzten Kraft für eine Unterfläche, die der ausgewählten Untergruppe entspricht, basierend auf den Daten, die durch die Untergruppe der Vielzahl von Sensoren gesammelt werden, konfiguriert ist, wobei bevorzugt die Untergruppe der Vielzahl von Sensoren basierend auf den Daten, die durch die Vielzahl von Sensoren gesammelt werden, ausgewählt wird und wobei weiter bevorzugt die Untergruppe der Vielzahl von Sensoren durch Vergleich der Daten, die durch jeden Sensor gesammelt werden, mit einem Referenzwert oder durch Vergleich der Daten, die durch jeden Sensor gesammelt werden, mit den Daten, die durch einen oder mehrere andere Sensoren gesammelt werden, ausgewählt wird und/oder wobei weiter bevorzugt der Prozessor zur Wiederholung der Berechnung der abgeschätzten Kraft konfiguriert ist und wobei Daten von der Untergruppe der Vielzahl von Sensoren bei höherer Rate gesammelt werden als Daten von Sensoren außerhalb der Untergruppe der Vielzahl von Sensoren gesammelt werden.

11. System nach einem der Ansprüche 8 bis 10, wobei der Prozessor zur Wiederholung der Berechnung der abgeschätzten Kraft unter Verwendung von Daten konfiguriert ist, die durch eine Vielzahl von Sensoren in ungleichmäßigen Zeitintervallen, bevorzugt in zufälligen oder pseudozufälligen Zeitintervallen gesammelt werden.

12. System nach einem der Ansprüche 8 bis 11, wobei das Sportpolster ein erstes Sportpolster ist und das weiter Folgendes umfasst: ein zweites Sportpolster nach einem der Ansprüche 1 bis 7 und einen Prozessor, der zur Berechnung einer abgeschätzten Kraft basierend auf den Daten, die durch eine Vielzahl von Sensoren des zweiten Sportpolsters gesammelt werden, konfiguriert ist, wobei der Prozessor oder die Prozessoren zum Vergleich der Daten, die durch die Vielzahl von Sensoren des ersten Sportpolsters gesammelt werden, mit den Daten, die durch die Vielzahl von Sensoren des zweiten Sportpolsters gesammelt werden, konfiguriert sind, um ein Ereignis, das zwischen den zwei Sportpolstern auftritt, basierend auf dem Vergleich zu identifizieren und eine Anzeige des Ereignisses, das zwischen den zwei Sportpolstern auftritt, auszugeben, wobei bevorzugt das Identifizieren des Ereignisses, das zwischen den zwei Sportpolstern auftritt, auf einem Vergleich der Größenordnung einer abgeschätzten Kraft des ersten Sportpolsters und der Größenordnung einer abgeschätzten Kraft des zweiten Sportpolsters und/oder einer Zeit der abgeschätzten Kraft des ersten Sportpolsters und einer Zeit der abgeschätzten Kraft des zweiten Sportpolsters basiert.

**13.** Verfahren zur Abschätzung einer Kraft, die durch ein äußeres Objekt auf ein Sportpolster ausgeübt wird, wobei das Verfahren Folgendes umfasst:

Sammeln (S100) unter Verwendung einer zweidimensionalen Reihe von Sensoren, wobei die Reihe mindestens 10 Sensoren umfasst, von Daten, die die Kraft, die auf jeden der jeweiligen Sensoren wirkt, anzeigen, wobei die Sensoren über ein Raster von Sensorpositionen innerhalb des Sportpolsters voneinander beabstandet sind und wobei jeder Sensor konfiguriert ist, um unabhängig Daten zu sammeln, die eine Kraft anzeigen;
Empfangen (S200), an einem Datenempfänger, der gesammelten Daten von jedem der Reihe von Sensoren;
Berechnen, unter Verwendung eines Prozessors, einer abgeschätzten Kraft basierend auf den Daten, die durch eine Vielzahl der Sensoren gesammelt werden;
**dadurch gekennzeichnet, dass**:

das Raster von Sensorpositionen ein ungleichmäßiges Raster von Sensorpositionen ist; und
jeder Sensor eine kraftempfindliche Fläche umfasst und wobei die gesamte kraftempfindliche Fläche der Reihe von Sensoren höchstens 50 % der Gesamtfläche beträgt, die durch die Reihe von Sensoren bedeckt ist.

**14.** Verfahren nach Anspruch 13, wobei der Schritt des Berechnens der abgeschätzten Kraft das Anpassen (S400) einer kontinuierlichen Funktion an die Daten, die durch die Vielzahl von Sensoren gesammelt werden, umfasst, wobei bevorzugt der Schritt des Berechnens der abgeschätzten Kraft das Berechnen (S500) eines Integrals der angepassten kontinuierlichen Funktion umfasst.

**15.** Verfahren nach einem der Ansprüche 13 bis 14, wobei das Berechnen der abgeschätzten Kraft auf den Daten, die durch eine Vielzahl der Sensoren gesammelt werden, basiert und auf einem vorgegebenen Abstand der Vielzahl von Sensoren basiert und/oder das Auswählen einer Untergruppe der Vielzahl von Sensoren und Berechnen einer abgeschätzten Kraft für eine Unterfläche, die der ausgewählten Untergruppe entspricht, basierend auf den Daten, die durch die Untergruppe der Vielzahl von Sensoren gesammelt werden, umfasst.

**Revendications**

**1.** Protection de sport (100) pour utilisation en estimant une force exercée par un objet externe sur la protection de sport, la protection de sport comprenant :

un réseau bidimensionnel de capteurs (104), le réseau de capteurs comprend au moins 10 capteurs, les capteurs étant espacés les uns des autres en travers d'une grille de positions de capteurs de la protection de sport, chaque capteur étant configuré pour recueillir indépendamment des données indicatives de la force agissant sur le capteur respectif ;
un récepteur de données (107) configuré pour recevoir les données recueillies de chacun du réseau de capteurs de sorte que les données d'une pluralité des capteurs peuvent être utilisées pour estimer une force,

**caractérisé en ce que** :

la grille de positions de capteurs est une grille non uniforme de positions de capteurs ; et
chaque capteur comprend une surface sensible à une force et dans laquelle la surface totale sensible à une force du réseau de capteurs fait au moins 50 % de la surface totale couverte par le réseau de capteur.

**2.** Protection de sport selon la revendication 1, dans laquelle les capteurs sont arrangés en travers d'une couche de substrat (108) de la protection de sport, dans laquelle de préférence la couche de substrat définit une gille, chaque capteur étant situé à une intersection de la grille définie par la couche de substrat.

**3.** Protection de sport selon l'une quelconque des revendications précédentes, dans laquelle les positions de capteurs sont arrangées de manière aléatoire ou pseudo-aléatoire en travers de la grille de positions de capteurs.

**4.** Protection de sport selon l'une quelconque des revendications précédentes, dans laquelle chaque capteur est configuré pour recueillir directement la force agissant sur le capteur respectif, et/ou dans laquelle chaque capteur comprend une surface sensible à une force d'au plus 25 cm², de préférence d'au plus 10 cm², plus préférentiellement d'au plus 5 cm², le plus préférentiellement d'au plus 1 cm².

**5.** Protection de sport selon l'une quelconque des revendications précédentes, comprenant en outre un transmetteur de données configuré pour transmettre les données reçues par le récepteur de données et étant configuré de préférence pour transmettre les données sans fil.

**6.** Protection de sport selon l'une quelconque des revendications précédentes, où la protection de sport est configurée pour être ajustée à une surface non plane de sorte que le réseau de capteurs définit une couche de détection non plane.

**7.** Protection de sport selon l'une quelconque des revendications précédentes, où la protection de sport est incorporée dans un article de sport tel qu'une armure, un vêtement de sport ou un équipement d'entraînement sportif, où la protection de sport est de préférence incorporée dans un article de sport vestimentaire, et dans laquelle le réseau de capteurs est arrangé de manière à s'étendre sur une zone correspondant à l'un ou plusieurs des épaules, bras, sternum, abdomen, côtés, haut du dos, milieu gauche du dos, milieu droit du dos et bas du dos du porteur.

**8.** Système configuré pour estimer une force exercée par un objet externe sur une protection de sport, le système comprenant :

une protection de sport selon l'une quelconque des revendications 1 à 7 ;
un processeur configuré pour calculer une force estimée sur la base des données recueillies par un pluralité de capteurs.

**9.** Système selon la revendication 8, dans lequel le processeur est configuré pour calculer une force estimée en ajustant une fonction continue aux données recueillies par la pluralité de capteurs, dans lequel le processeur est configuré de préférence pour calculer une force estimée en calculant l'intégrale de la fonction continue ajustée.

**10.** Système selon l'une quelconque des revendications 8 à 9, dans lequel le processeur est configuré pour sélectionner des données d'un sous-ensemble de la pluralité de capteurs et calculer une force estimée pour une sous-zone correspondant au sous-ensemble sélectionné sur la base des données recueillies par ledit sous-ensemble de la pluralité de capteurs, dans lequel le sous-ensemble de la pluralité de capteurs est sélectionné de préférence sur la base des données recueillies par la pluralité de capteurs, et dans lequel de préférence encore le sous-ensemble de la pluralité de capteurs est sélectionné en comparant les données recueillies par chaque capteur à une valeur de référence, ou en comparant les données recueillies par chaque capteur aux données recueillies par un ou plusieurs autres capteurs, et/ou dans lequel de préférence encore le processeur est configuré pour répéter le calcul de la force estimée et dans lequel les données sont recueillies du sous-ensemble de la pluralité de capteurs à une cadence plus rapide que les données recueillies de capteurs qui ne sont pas dans le sous-ensemble de la pluralité de capteurs.

**11.** Système selon l'une quelconque des revendications 8 à 10, dans lequel le processeur est configuré pour répéter le calcul de force estimée en utilisant des données recueillies par une pluralité de capteurs à des intervalles de temps non uniformes, de préférence à des intervalles de temps aléatoires ou pseudo-aléatoires.

**12.** Système selon l'une quelconque des revendications 8 à 11, dans lequel la protection de sport est une première protection de sport et comprenant en outre une deuxième protection de sport conformément à l'une quelconque des revendications 1 à 7 et un processeur configuré pour calculer une force estimée sur la base des données recueillies par une pluralité des capteurs de la deuxième protection de sport, le processeur ou des processeurs étant configurés pour comparer les données recueillies par la pluralité de capteurs de la première protection de sport aux données recueillies par la pluralité de capteurs de la deuxième protection de sport, afin d'identifier un événement survenant entre les deux protections de sport sur la base de ladite comparaison, et sortir une indication de l'événement survenant entre les deux protections de sport, dans lequel de préférence identifier l'événement survenant entre les deux protections de sport est basé sur une comparaison de la grandeur d'une force estimée de la première protection de sport et de la grandeur d'une force estimée de la deuxième protection de sport et/ou d'un moment de la force estimée de la première protection de sport et d'un moment de la force estimée de la deuxième protection de sport.

**13.** Procédé d'estimation d'une force exercée par un objet externe sur une protection de sport, le procédé comprenant :

recueillir (S100), en utilisant un réseau bidimensionnel de capteurs, le réseau comprenant au moins 10 capteurs, des données indicatives de la force agissant sur chacun des capteurs respectifs, dans lequel les capteurs sont espacés les uns des autres en travers d'une grille de positions de capteurs dans la protection de sport, et dans lequel chaque capteur est configuré pour recueillir indépendamment les données indicatives de force ; recevoir (S200), à un récepteur de données, les données recueillies de chacun du réseau de capteurs ; calculer, en utilisant un processeur, une force estimée sur la base des données recueillies par une pluralité des capteurs ;

**caractérisé en ce que** :

la grille de positions de capteurs est une grille non uniforme de positions de capteurs ; et
chaque capteur comprend une surface sensible à une force et dans lequel la surface totale sensible à une force du réseau de capteurs fait au plus 50 % de la surface totale couverte par le réseau de capteurs.

**14.** Procédé selon la revendication 13, dans lequel l'étape consistant à calculer la force estimée comprend ajuster (S400) une fonction continue aux données recueillies par la pluralité de capteurs, dans lequel de préférence l'étape de calcul de la force estimée comprend calculer (S500) une intégrale de la fonction continue ajustée.

**15.** Procédé selon l'une quelconque des revendications 13 à 14, dans lequel calculer la force estimée est basé sur les données recueillies par un pluralité des capteurs et basé sur un espacement prédéterminé de la pluralité de capteurs et/ou comprenant sélectionner un sous-ensemble de la pluralité de capteurs et calculer une force estimée pour une sous-zone correspondant au sous-ensemble sélectionné sur la base des données recueillies par ledit sous-ensemble de la pluralité de capteurs.

# Fig. 1

# Fig. 2A

# Fig. 2B

# Fig. 2C

# Fig. 3

104 104 104 104 104 104 104

105b 105b 105b 105b 105b 105b 105b

107

100

111

110

# Fig. 4

Collect pressure data using each pressure sensor of an array of pressure sensors. — S100

S200 — Receive the collected pressure data at an electronics unit.

Transmit the collected pressure data to the external electronics unit. — S300

S400 — Fit a continuous function to the data collected by the plurality of pressure sensors using a processor.

Calculate the integral of the fitted continuous function using the processor. — S500

S600 — Output an estimated force of impact based on the calculation of the integral of the fitted continuous function.

**EP 3 906 098 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2549463 A
- US 2016338644 A1
- US 2011260890 A1
- US 2017156667 A1